Europäisches Patentamt

European Patent Office

Office européen des brevets

(19)

(11) **EP 0 874 612 B2**

(12) **NEW EUROPEAN PATENT SPECIFICATION**

(45) Date of publication and mention
of the opposition decision:
**07.12.2005 Bulletin 2005/49**

(45) Mention of the grant of the patent:
**21.08.2002 Bulletin 2002/34**

(21) Application number: **96945289.5**

(22) Date of filing: **19.12.1996**

(51) Int Cl.⁷: **A61F 13/15**

(86) International application number:
**PCT/US1996/020683**

(87) International publication number:
**WO 1997/024095 (10.07.1997 Gazette 1997/30)**

(54) **ABSORBENT ARTICLES HAVING FLUID CONTACT ANGLE GRADIENTS AND APERTURED BACKSHEET LAYER**

ABSORBIERENDE ARTIKEL MIT FLÜSSIGKEITSKONTAKTWINKELGRADIENTEN UND PERFORIERTE, ÄUSSERE SCHICHT

ARTICLE ABSORBANT PRESENTANT DES GRADIENTS D'ANGLE DE CONTACT AVEC DES FLUIDES, ET POURVU D'UNE COUCHE POSTERIEURE PERFOREE

(84) Designated Contracting States:
**AT BE CH DE DK ES FI FR GB GR IE IT LI LU NL PT SE**

(30) Priority: **28.12.1995 EP 95120647**

(43) Date of publication of application:
**04.11.1998 Bulletin 1998/45**

(73) Proprietor: **THE PROCTER & GAMBLE COMPANY**
**Cincinnati, Ohio 45202 (US)**

(72) Inventors:
• **BEWICK-SONNTAG, Christopher, Philip**
**I-65125 Pescara (IT)**
• **VEGLIO, Paolo**
**I-65100 Pescara (IT)**

(74) Representative:
**Kremer, Véronique Marie Joséphine et al**
**Procter & Gamble Service GmbH**
**Sulzbacher Strasse 40-50**
**65824 Schwalbach am Taunus (DE)**

(56) References cited:
| EP-A- 0 040 084 | EP-A- 0 104 906 |
| EP-A- 0 272 118 | EP-A- 0 481 322 |
| EP-A- 0 596 532 | EP-A- 0 674 892 |
| EP-A- 0 705 584 | WO-A-94/22408 |
| WO-A-95/16562 | CA-A- 1 033 903 |
| GB-A- 2 266 465 | US-A- 3 881 489 |
| US-A- 4 306 559 | US-A- 5 334 177 |

**EP 0 874 612 B2**

**Description**

Field of the Invention

The present invention relates to absorbent article in particular sanitary napkins having a breathable backsheet which exhibit reduced wet through onto the users garments.

Background of the Invention

The primary consumer needs which underlie development in the absorbent article field, in particular catamenials is a high protection and comfort level.

One highly desirable means of improving the comfort of absorbent articles is the use of so called 'breathable backsheets'. One type of breathable backsheet is a 2 dimensional or planar micropororus film. Such breathable backsheets are primarily vapour permeable allowing gaseous exchange with the environment. This thereby allows for the evaporation of a portion of the fluid stored in the core and increases the circulation of air within the absorbent article. This is particularly beneficial as it reduces the sticky feeling experienced by many wearers during use, particularly over extended periods of time. Obviously the larger the apertures the better the permeability of the layer.

However, the main drawback associated with the use of breathable backsheets in absorbent article is the increased probability of leakage, commonly referred to as wet through onto the users garment. Although such breathable backsheets are intended in principle only to allow the transfer of materials in the gaseous state physical mechanisms such as extrusion, diffusion and capillary transport may still occur and result in the transfer of the fluids through the backsheets and onto the users garments. In particular, these mechanisms become more dominant if the product is utilised during physical exertion, for heavy loads or over extended periods of time. In effect, whilst breathable backsheets provide excellent comfort improvements they result in an unacceptable level of failure with regard to protection, especially under stressed conditions.

The problem of wet through onto user's garments due to the incorporation of such breathable backsheets in absorbent articles has been recognized in the art. Attempts to solve the problem have mainly resided in the use of multiple layer backsheet such as those illustrated in US 4 341 216. Similarly EP 710 472 discloses breathable absorbent articles comprising a breathable backsheet consisting of at least two breathable layers which are unattached to one another over the core area. Also EP 710 471 discloses a breathable backsheet for disposable absorbent articles comprising an outer layer of a gas permeable, hydrophobic, polymeric fibrous fabric and an inner layer comprising an apertured formed film having directional fluid transport. US 3 881 489 discloses a breathable backsheet comprising two layers having a gradient in void volume. EP 710472 discloses a breathable backsheet comprising at least two layers which are essentially non-attached to each other. The inner layer preferably comprises a film with funnel-shaped apertures having directional fluid transport.

However, none of the above solutions have proved fully satisfactory. This is particularly the case for thin products, as thickness is also considered as a key variable affecting product comfort. Thus, there exists a dichotomy in the methods available to provide increased comfort absorbent products, such that thin breathable products cannot provide the desired level of protection.

As a result, there exists a need to provide an absorbent article which offers improved comfort by the employment of a breathable backsheet and having a reduced thickness which maintains the required level of protection.

It has now been found that breathable backsheets may be utilised in thin sanitary napkins, thereby providing both a high level of protection and comfort by creating a hydrophobicity gradient between the backsheet and the core, achieved by the utilisation of a low surface energy materials such as silicone or chlorofluorocarbons or a low surface energy treatment. In this manner it is believed that the physical mechanisms such as capillary and diffusion transport are hindered and wet through is considerably reduced if not completely eliminated.

The use of surface energy gradients as such is discussed in EP 767 648. It discloses fluid transport webs e. g. topsheets which exhibit surface energy gradients. The web facilitates fluid transport in one direction and resists transport in the opposite direction. The web comprises first and second surfaces, which are separated from one another by an intermediate portion. The first surface of the web has a lower surface energy than the surface energy of the intermediate, thereby creating a surface energy gradient. Suitable low surface energy materials include silicone, fluoropolymers and paraffins. The web is particularly suited as a topsheet for absorbent articles in order to transport fluid away from the wearer-contacting surface. US 5 334 177 discloses the utilization of a gradient in liquid-solid contact angle in the core of absorbent articles for the distribution of liquid from a zone of vulnerability, situated in a region of high potential exposure to initial wetting, to an additional core zone which are in fluid contact with the zone of vulnerability. The liquid-solid contact angle is decreasing from the zone of vulnerability towards the additional core zone.

## Summary of the Invention

The present invention relates to a disposable absorbent article comprising a liquid pervious topsheet, an absorbent core and a backsheet. The core is intermediate the topsheet and the backsheet and the backsheet comprises a gas permeable 2-dimensional apertured layer and the core comprises a fluid storage layer. The backsheet comprises an outer layer. The core and the backsheet each comprise at least one layer, wherein each layer has a wearer facing surface and a garment facing surface and each of said surfaces of said layers has a fluid contact angle. The absorbent article has a lower portion extending from and including the garment facing surface of the fluid storage layer to and including the garment facing surface of the outer layer. The wearer facing surface of at least one of the layers in the lower portion has a fluid contact angle greater than the fluid contact angle of the adjacent garment facing surface of an adjacent layer.

Moreover the garment facing surface of at least one of said layers in the lower portion has a fluid contact angle greater than the fluid contact angle of the wearer facing surface of said same layer.

A further aspect of the present invention relates to a process for the production of an absorbent article described above comprising the step of applying a low surface energy material to the surface of at least one of the layers in the lower portion.

## Brief Description of the Drawings

Fig. 1: Top plan view of a first embodiment of an absorbent article of the present invention with portions cut away to show its construction.

Fig. 2: Enlarged cross sectional view of a backsheet of the present invention taken along line I-I of Fig. 1.

Fig. 3: An enlarged cross sectional view of a droplet of liquid on a surface, where angle A illustrates the contact angle of the liquid with the surface.

Fig. 4: An enlarged cross sectional view of a droplet of liquid on a surface having two different surface energies, thus exhibiting two different contact angles A(a) and A(b).

## Detailed Description of the Invention

The present invention relates to absorbent disposable articles such as sanitary napkins (1), baby diapers, incontinence products and panty liners. Typically such products comprise a liquid pervious topsheet (2), a backsheet (3) and an absorbent core (4) intermediate said topsheet (2) and said backsheet (3). The topsheet (2), backsheet (3) and core (4) each have a wearer facing surface and a garment facing surface. The garment facing surface of the topsheet and the wearer facing surface of the backsheet are joined to one another at the periphery (5) of said absorbent article. As used herein the term lower portion refers to the portion of the absorbent article extending from and including the garment facing surface of said fluid storage layer to and including the garment facing surface of said outer layer. In a preferred embodiment of the present invention the absorbent article has wings, side wrappings or sideflaps.

## Backsheet

The absorbent articles according to the present invention comprise a breathable backsheet (20). The backsheet (20) primarily prevents the extrudes absorbed and contained in the absorbent structure from wetting articles that contact the absorbent product such as underpants, pants, pyjamas and undergarments. In addition however, the backsheet of the present invention permits the transfer of both vapour and to some extent air through it and thus allows the circulation of air into and out of the backsheet.

According to the present invention the backsheet comprises at least one layer comprising a gas permeable, 2-dimensional substantially planar, apertured layer (21). As used herein the term 2-dimensional substantially planar refers to layers having a thickness of less than 1mm, preferably less than 0.5mm, wherein the apertures are all within the plane of the layer. Thus, as used herein the term 2 dimenional layer does not include apertured preformed films having apertures which protrude out of the plane of the layer.

According to the present invention the apertures in said 2 dimensional layer may be of any configuration, but are preferably circular or oblong. The apertures may also be of varying dimensions. Typically, the apertures have average diameters of from 150 micrometers to 5 micrometers, preferably from 120 micrometers to 5 micrometers, most preferably from 90 micrometers to 5 micrometers. Preferably the entire surface of the 2 dimensional backsheet has apertures which are evenly distributed throughout the entire surface area. However, backsheets having only certain regions of the surface area comprising apertures such as the central portion or the peripheral portion is also envisioned to be within the scope of the present invention.

The 2 dimensional apertured layer of the backsheet may be made of any material known in the art, but is preferably manufactured from commonly available polymeric materials such as polyethylene or polypropylene. Suitable microporous material include XMP-1001 of Minnesota Mining and Manufacturing Company, St. Paul, Minosota, USA and XBF-100W available from Exxon Chemicals, Illinios, USA. Suitable materials are for example Gortex (TM) or Sympatex (TM) type materials well known in the art for there application in so-called breathable clothing. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e stretching the layer).

According to the present invention the backsheet may comprise in addition to said 2-dimensional layer, further layers, preferably at least one additional layer (22). The additional layers may be comprised of any material in the art which is gas permeable. Preferably the backsheet of the present invention comprises at least one layer selected from wovens, nonwovens, formed apertured polymeric films, preferably having protruding apertures to facilitate fluid transport from the backsheet to the core or 2-dimensional apertured layers similar to the first layer described herein above. Preferably the second layer of said backsheet comprises either a one directional fluid transport apertured layer or a fibrous fabric layer composed of polymeric fibres such as polymeric non wovens known in the art The fibrous fibre layer preferably has a basis weight of 10 to 100g/m$^2$, more preferably 15 to 30g/m$^2$. The fibres can be made of any polymeric material, in particular, fibres of polyethylene, polypropylene, polyester polyacetate or combinations thereof (inter- and infra fibre combinations). Also mixtures of synthetic fibres and non absorbent natural fibres or treated natural fibres such as cotton may be utilised for the second layer. The fibres are preferably spunbonded, carded or melt blown. Preferably the second layer comprises a matrix of spunbonded fibres covered on one sides with meltblown fibres or alterntively a matrix of meltblown fibres covered on both sides with spun blown fibres. The second layer of the backsheet may in addition comprise at least 5% by weight of said layer of fibres which are liquid absorptive such that the fibres swell and reduce inter-fibre spacing.

According to the present invention the backsheet is located adjacent and below the core extending towards the garment facing surface of the absorbent article. If the backsheet comprises only one layer that being the 2-dimensional layer, this layer will be adjacent the core. However in the case that the backsheet comprises multiple layers the 2 dimensional layer may be positioned either above or below said layers and thus may not be located immediately adjacent the core. All of the layers comprising the backsheet are substantially in intimate and direct contact with one another.

The backsheet typically extends across the whole of the absorbent structure and can extend into and form part of or all sideflaps, side wrapping elements or wings.

Absorbent core

According to the present invention, the absorbent core (23) comprises a first portion and a second portion, said first portion may comprise the following components: (a) an optional primary fluid distribution layer preferably together with a secondary optional fluid distribution layer; (b) a fluid storage layer; and said second portion may comprise (c) an optional fibrous ("dusting") layer underlying the storage layer; and (d) other optional components. According to the present invention the absorbent core may have any thickness depending on the end use envisioned. In a preferred embodiment of the present invention wherein the absorbent article is a sanitary napkin or a panty liner, the core may have a thickness of from 15mm to 1mm, preferably from 10mm to 1mm, most preferably from 7mm to 1mm.

a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers.

b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bonds. Absorbent gelling material or other optional material can be comprised between the layers.

Modified cellulose fibres such as the stiffened cellulose fibers can also be used. Synthetic fibres can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibres, tricomponent fibres, mixtures thereof and the like. Preferably, the fibre surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.


c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.


d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.


The topsheet

The topsheet (24) may comprise a single layer or a multiplicity of layers. In a preferred embodiment the topsheet comprises a first layer (25) which provides the user facing surface of the topsheet and a second layer (26) between the first layer and the absorbent core (23).

The topsheet as a whole and hence each layer individually needs to be compliant, soft feeling, and nonirritating to the wearer's skin. It also can have elastic characteristics allowing it to be stretched in one or two directions. According to the present invention the topsheet may be formed from any of the materials available for this purpose and known in the art, such as non woven fabrics, films or combinations of both. In a preferred embodiment of the present invention at least one of the layers (preferably the upper layer) of the topsheet comprises a liquid permeable apertured polymeric film (25).

Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523.

The topsheet typically extends across the whole of the absorbent structure and can extend into and form part of or all of the preferred sideflaps, side wrapping elements or wings.

Fluid contact angle

According to the present invention any layer in said lower portion has a wearer facing surface and a garment facing surface and each of said surfaces has a fluid contact angle, wherein the wearer facing surface of at least one of said layers in said lower portion has a fluid contact angle greater than the fluid contact angle of the garment facing surface of the adjacent garment facing surface of an adjacent layer.

Any layer in lower portion has a wearer facing surface and a garment facing surface and each of said surfaces of said layers has a fluid contact angle wherein the garment facing surface of at least one of said layers in said lower portion has a fluid contact angle greater than the fluid contact angle of the wearer facing surface of said same layer.

In principle the contact angle gradient may be present in said lower portion between any surface (wearer facing or garment facing) of any layer therein. Thus, the fluid contact angle gradient may be present across the wearer and garment facing surface of the same layer or between the garment facing surface of at least one layer in said lower portion and an adjacent surface of an adjacent layer, i.e. between the wearer and the garment facing surface of the first layer of the backsheet, between the garment facing surface of the first layer and the wearer facing surface of the second layer of the backsheet, between the wearer and the garment facing surface of the second layer of the backsheet or between any subsequent backsheet layer. In addition, it is also foreseen that combinations of these layers each exhibiting a specific contact angle relation be utilised thereby, producing a continuous gradient in contact angles in said lower portion.

However, for simplicity purposes the description of the invention hereinafter will focus on the presence of a distinct or increased contact angle gradient between the garment facing surface of the core and the wearer facing surface of the first layer of the backsheet.

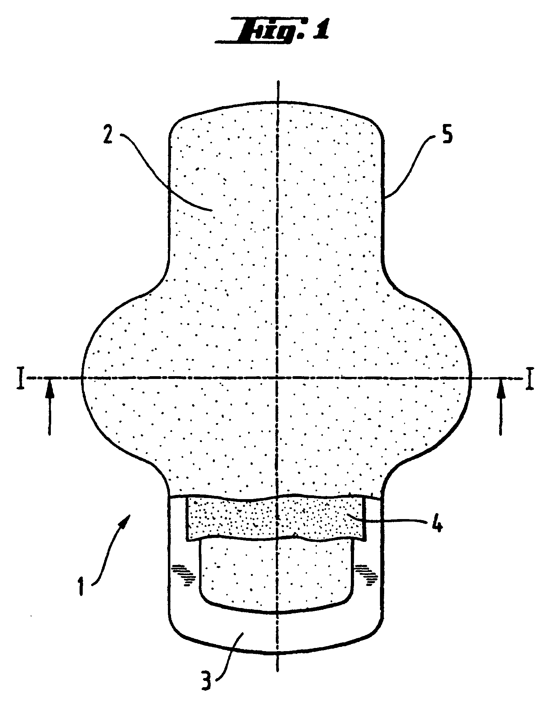

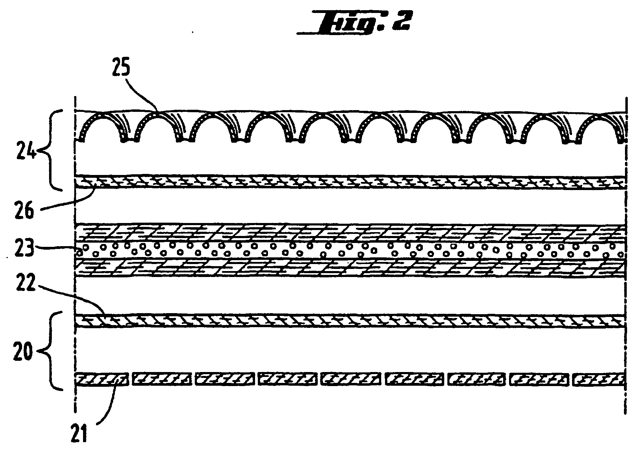

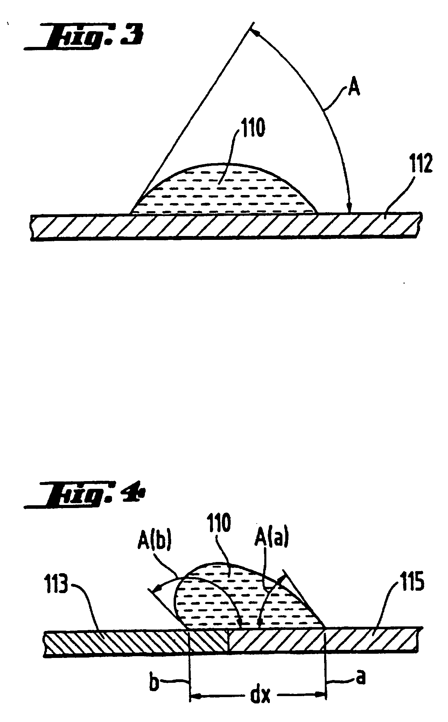

Typically, a drop of liquid 110 placed on a solid surface 112 makes a contact angle, A, with the solid surface, as seen in Fig. 3. As the wettability of the solid surface by the liquid increases, the contact angle, A, decreases. As the wettability of the solid surface by the liquid decreases, the contact angle, A, increases. The liquid-solid contact angle may be determined from techniques known in the art, such as those described in greater detail in Physical Chemistry of Surfaces, Second Edition, by Arthur W. Adamson (1967), F. E. Bartell and H. H. Zuidema, J. Am. Chem. Soc., 58, 1449 (1936), and J. J. Bikerman, Ind. Eng. Chem., Anal. Ed., 13, 443 (1941). More recent publications in this area include Cheng, et al., Colloids and Surfaces 43:151-167 (1990), and Rotenberg, et al., Journal of Colloid and Interface Science 93(1):169-183 (1983).

As used herein, the term "hydrophilic" is used to refer to surfaces that are wettable by aqueous fluids (e.g., aqueous body fluids) deposited thereon. Hydrophilicity and wettability are typically defined in terms of contact angle and the surface tension of the fluids and solid surfaces involved. This is discussed in detail in the American Chemical Society publication entitled Contact Angle, Wettability and Adhesion, edited by Robert F. Gould (Copyright 1964) A surface is said to be wetted by an aqueous fluid (hydrophilic) when the fluid tends to spread spontaneously across the surface. Conversely, a surface is considered to be "hydrophobic" if the aqueous fluid does not tend to spread spontaneously across the surface.

The fluid contact angle depends on surface inhomogeneities (e.g., chemical and physical properties, such as roughness), contamination, chemical/physical treatment of or composition of the solid surface, as well as the nature of the liquid and its contamination. The surface energy of the solid also influences the contact angle. As the surface energy of the solid decreases, the contact angle increases. As the surface energy of the solid increases, the contact angle decreases.

The energy required to separate a liquid from a solid surface (e.g., a film or fiber) is expressed by equation (1):

$$(1) \qquad W = G (1 + \cos A)$$

where:

W is the work of adhesion measured in $erg/_{cm}2$, $(x10^{-3}Jm^{-2})$
G is the surface tension of the liquid measured in dyne/cm, $(x10^3Nm^{-1})$ and
A is the liquid-solid contact angle measured in degrees.

For a given liquid, the work of adhesion increases with the cosine of the liquid-solid contact angle (reaching a maximum where the contact angle A is zero).

Work of adhesion is one useful tool in understanding and quantifying the surface energy characteristics of a given surface for a given liquid.

Table 1 is useful to illustrate the relationship between solid-liquid contact angle and work of adhesion for a particular fluid (e.g., water), whose surface tension is 75 dynes/cm $(75x10^{-3}Jm^{-2})$.

TABLE 1

| A (degrees) | cos A | 1+cos A | W ($erg/cm^2$ $(x10^{-3}$ $Jm^{-2})$ |
|---|---|---|---|
| 0 | 1 | 2 | 150 |
| 30 | 0.87 | 1.87 | 140 |
| 60 | 0.5 | 1.50 | 113 |
| 90 | 0 | 1.00 | 75 |
| 120 | -0.5 | 0.5 | 38 |
| 150 | -0.87 | 0.13 | 10 |
| 180 | -1 | 0 | 0 |

As depicted in Table 1, as the work of adhesion of a particular surface decreases (exhibiting a lower surface energy of the particular surface), the contact angle of the fluid on the surface increases, and hence the fluid tends to "bead up" and occupy a smaller surface area of contact. The reverse is likewise true as the surface energy of a given surface decreases with a given fluid. The work of adhesion, therefore, influences interfacial fluid phenomena on the solid surface.

More importantly, in the context of the present invention, surface energy gradients as illustrated by fluid contact angles or discontinuities have been found to be useful in preventing fluid transport. Fig. 4 illustrates a droplet of fluid 110 which is located on a solid surface having two regions 113 and 115 having differing surface energies (indicated by the different cross-hatching for illustrative purposes). In the situation illustrated in Fig. 4, region 113 exhibits a comparatively lower surface energy than region 115, and hence a reduced wettability for the fluid of the droplet than region 115. Accordingly, the droplet 110 produces a contact angle A(b) at the edge of the droplet contacting region 113 which is greater than the contact angle A(a) produced at the edge of the droplet contacting region 115. It should be noted that although for graphic clarity the points "a" and "b" lie in a plane, the distance "dx" between points "a" and "b" need not be linear, instead representing the extent of droplet/surface contact regardless of the shape of the surface. Droplet 110 thus experiences a surface energy imbalance and hence an external force due to the differences in the relative surface energies (i.e., the surface energy gradient or discontinuity) between regions 113 and 115, which can be represented by the equation (2):

$$(2) \qquad dF = G [\cos A(a) - \cos A(b)] \, dx$$

where:

dF is the net force on the fluid droplet,
dx is the distance between the reference locations "a" and "b",
G is as defined previously, and
A(a), and A(b) are the contact angles A at locations "a" and "b", respectively.

Solving equation (1) for cos A(a) and cos A(b) and substituting into equation (2) yields equation (3):

$$(3) \qquad dF = G[(W(a)/G - 1) - (W(b)/G - 1)] \, dx$$

Equation (3) can be simplified to equation (4):

$$(4) \qquad dF = (W(a) - W(b)) \, dx$$

The importance of the differential in surface energy between the two surfaces is clearly depicted in equation (4), as is the directly proportional effect that changes in the magnitude of the differential in work of adhesion would have on the magnitude of the force.

More detailed discussions of the physical nature of surface energy effects and capillarity may be found in Textile Science and Technology; Volume 7, Absorbency, edited by Portnoy K. Chatterjee (1985), and Capillarity, Theory and Practice, Ind. Eng. Chem. 61,10 (1969) by A. M. Schwartz.

Accordingly, the force experienced by a droplet will cause movement in the direction of the surface featuring the higher surface energy in this case towards the core. For simplicity and graphic clarity, the surface energy gradient or discontinuity has been depicted in Fig. 4 as a single, sharp discontinuity or boundary between well-defined regions of constant but differing surface energy. Surface energy gradients may also exist as a continuous gradient or a stepwise gradient, with the force exerted on any particular droplet (or portions of such droplet) being determined by the surface energy at each particular area of droplet contact.

As used herein, the term "gradient" when applied to differences in surface energy or work of adhesion is intended to describe a change in surface energy or work of adhesion occurring over a measurable distance. The term "discontinuity" is intended to refer to a type of "gradient" or transition, wherein the change in surface energy occurs over an essentially zero distance. Accordingly, as used herein all "discontinuities" fall within the definition of "gradient".

Also, as used herein the terms "capillary" and "capillarity" are used to refer to passageways, apertures, pores, or spaces within a structure which are capable of fluid transport in accordance with the principles of capillarity generally represented by the Laplace equation (5):

$$(5) \qquad p = 2G \, (\cos A)/R$$

where:

p is the capillary pressure;
R is the internal radius of the capillary (capillary radius); and
G and A are as defined above.

As noted in Penetration of Fabrics by Emery I. Valko, found in Chapter III of Chem. Aftertreat. Text. (1971), pp. 83-113, for A = 90°, the cosine of A is zero and there is no capillary pressure. For A > 90°, the cosine of A is negative and the capillary pressure opposes the entry of fluid into the capillary. Hence, for hydrophilic aqueous liquids the capillary walls should be of a hydrophilic nature for an appreciable capillary phenomena to occur. Also, R must be sufficiently small for p to have a meaningful value, since as R increases (larger aperture/capillary structure) the capillary pressure decreases.

Perhaps at least as important as the presence of surface energy gradients is the particular orientation or location of the gradients themselves with respect to the orientation and location of the capillaries or fluid passageways themselves.

Water is used as a reference liquid throughout only as an example for discussion purposes, and is not meant to be limiting. The physical properties of water are well-established, and water is readily available and has generally uniform properties wherever obtained. The concepts regarding work of adhesion with respect to water can easily be applied to other fluids such as blood, menses and urine, by taking into account the particular surface tension characteristics of the desired fluid.

By having a surface energy gradient between the core and backsheet creating a relatively low surface energy adjacent the portion of the backsheet which will be placed adjacent to and in contact with the absorbent core and a relatively lower surface energy portion located towards contact with the wearer's skin, the backsheet will be capable of hindering the movement of a drop of liquid from the core exhibiting the relatively higher surface energy to the backsheet exhibiting the relatively lower surface energy. The motion of the drop of liquid is induced by the contact angle differential between the lower surface energy portion and the higher surface energy portion which results in an imbalance in surface tension force acting on the solid-liquid contact plane. It is believed that the resulting surface energy gradient, which results in a negative capillary pressure is particularly suited for use with an apertured backsheet on an absorbent article.

The potential for wet through is thereby reduced by having an apertured backsheet with a surface energy gradient according to the aforementioned description. As some in-use forces tend to force the collected fluid to be squeezed out of the pad (e.g., squeezed by compression from the absorbent core towards the lower surface of the backsheet), such undesirable movement will be resisted by the surface of the backsheet which has a relatively low surface energy to repel the fluid as it attempts to make its way out of the pad through the openings in the backsheet.

Thus, the fluid is more readily retained in the absorbent core due to the driving forces of the surface energy gradients between the core and at least one of the layers of the backsheet.

With regard to the surface energy gradients of the present invention, it is important to remember that the upper and lower bounds of any such gradient are relative with respect to one another, i.e., the regions of the backsheet and core whose interface defines a surface energy gradient need not be on different sides of the hydrophobic/hydrophilic spectrum. That is to say, a gradient may be established by two surfaces of diverse degrees of hydrophobicity or diverse degrees of hydrophilicity, and need not necessarily be established with regard to a hydrophobic surface and a hydrophilic surface. Notwithstanding the foregoing, it is presently preferred that the upper surface of the backsheet have a comparatively low surface energy, i.e., that it be generally hydrophobic, in order to maximize the driving force imparted to the incoming fluid from the core and minimize the overall wet through of the backsheet on the garment-contacting surface.

Accordingly, in the present invention the surface energy gradients provide a synergistic effect in combination with the 2 dimensional layer backsheet to prevent fluid transport through the backsheet. Fluid on the first surface of the backsheet encounters two differing, but complementary driving forces which oppose its motion away from the core to the backsheet and towards the garment. These two forces likewise combine to oppose fluid movement toward the backsheet, thus dramatically reducing the incidence of wet through.

A number of physical parameters should be considered in designing an apertured backsheet and a core according to the absorbent article of the present invention, more particularly with regard to appropriately sizing and positioning the surface energy gradients for proper fluid handling. Such factors include the magnitude of the surface energy differential (which depends upon the materials utilized), migratability of materials, biocompatibility of materials, porosity or capillary size, overall caliper and geometry, fluid viscosity and surface tension, and the presence or absence of other structures on either side of the interfaces.

Preferably the difference in fluid contact angle between two adjacent surfaces in said lower portion providing a surface energy gradient should be at least 10°, preferably at least 20° and the surface having the lower surface energy should have a fluid contact angle of at least 90°, preferably at least 100°, more preferably at least 110°, most preferably at least 120°.

Backsheets according to the present invention may be prepared by any of the methods known in the art and as described for example in US 4, 777, 073 and is then rendered hydrohilic by means such as a corona discharge treatment generally in accordance with the teachings of U.S. Pat. Nos. 4,351,784 issued to Thomas et al. on Sept. 28, 1982; 4,456,570 issued to Thomas et al. on Jun. 26, 1984; and 4,535,020 issued to Thomas et al. on Aug. 13, 1985. A surface treatment having a relatively lower surface energy is then applied to the wearer facing surface of the apertured layer and is preferably cured. A suitable surface treatment is a silicone release coating from Dow Coming of Midland, Michigan available as Syl-Off 7677 to which a crosslinker available as Syt-Off 7048 is added in proportions by weight of 100 parts to 10 parts, respectively. Another suitable surface treatment is a coating of a UV curable silicone comprising a blend of two silicones commercially available from General Electric Company, Silicone Products Division, of Waterford, NY, under the designations UV 9300 and UV 9380C-D1, in proportions by weight of 100 parts to 2.5 parts, respectively. Typically low surface energy materials utilised at levels of at least 0.25g preferably 0.5 to 8.0 grams per square meter of surface area have performed satisfactorily, although other coating levels may prove suitable for certain applications depending upon the nature of the backsheet and the characteristics of the fluid, etc. Due to the planar nature of the backsheet layer the application of the low surface energy treatment must be carried out such that the apertures are not blocked.

Other suitable treatment materials include, but are not limited to, fluorinated materials such as fluoropolymers (e.g., polytetrafluoro- ethylene (PTFE), commercially available under the trade name TEFLON") and chlorofluoropolymers. Other materials which may prove suitable for reduced surface energy include hydrocarbons such as petrolatum, latexes, paraffins, and the like, although silicone materials are presently preferred for use in the absorbent article context for their biocompatibility properties. As used herein, the term "biocompatible" is used to refer to materials having a low level of specific adsorption for, or in other words a low affinity for, bio-species or biological materials such as gluco-proteins, blood platelets, and the like. As such, these materials tend to resist deposition of biological matter to a greater extent than other materials under in-use conditions. This property enables them to better retain their surface energy properties as needed for subsequent fluid handling situations. In the absence of biocompatibility, the deposition of such biological material tends to increase the roughness or non-uniformity of the surface, leading to increased drag force or resistance to fluid movement. Consequently, biocompatibility corresponds to reduced drag force or resistance to fluid movement, and hence faster access of fluid to the surface energy gradient and capillary structure. Maintenance of substantially the same surface energy also maintains the original surface energy differential for subsequent or enduring fluid depositions.

Biocompatibility, however, is not synonymous with low surface energy. Some materials, such as polyurethane, exhibit biocompatibility to some degree but also exhibit a comparatively high surface energy. Presently preferred materials such as silicone and fluorinated materials advantageously exhibit both low surface energy and biocompatibility.

According to the present invention the absorbent article is constructed by joining the various elements such as topsheet, backsheet and absorbent core by any means well known in the art. For example the backsheet and/or topsheet may be joined to the absorbent core or to each other by a uniform continuous layer of adhesive, a patterned layer of adhesive, or any array of separate lines, spirals or spots of adhesive. Alternatively, the elements may be joined by heat bonds, pressure bonds, ultra sonic bonds, dynamic mechanical bonds or any other suitable joining means known in the art and any combination thereof.

According to the present invention the absorbent article may find utility as sanitary napkins, panty liners, adult incontinence products and baby diapers. The present invention finds particular susceptibility as sanitary napkins and panty liner. Thus in addition to the components described herein above, the absorbent article may also comprise all those features and parts which are typical for products in the context of their intended use such as wings and side flaps, undergarment adhesive means and release paper, wrapping elements, fastening means and the like.

TEST METHODS

Method Nr. 1a & 1b -Wet-Through Test

The wet-through test is utilised to evaluate the resistance of a breathable backsheet or backsheet construction to transmission of bodily discharges. It can be used as a direct measure of how liquid-impervious the breathable backsheet is to the full range of bodily discharges by simply changing the composition of the test solution.

Basic Principle of the Methods:

The basic principle of the test is to simulate the loading of a disposable absorbent article in-use with bodily discharges. To achieve this a product is prepared, for example a sanitary napkin, and placed flat on a transparent test stand made of perspex. The product is oriented with the wearer facing side exposed (upper side) and the garment facing side in contact with the test stand (bottom side). Suspended above the sample to be analysed is a liquid delivery system that is capable of delivering any desired quantity of the desired test liquid (either as a burst or as a series of steps as is desired).

Located between the back most surface of the test sample and the see through test stand is a sheet of absorbent filter paper {produced by Cartiera Favini S.p.A. Italy; Type Abssorbente Bianca "N30" (local vendor Ditta Bragiola SpA. Perugia, Italy)}. This absorbent filter paper is in intimate contact with the backsheet of the test sample to simulate, for example a sanitary napkin attached to a panty or a diaper/incontinence device in close contact with the clothing. Directly below the transparent test stand is a mirror so positioned to allow any change in the absorbent filter paper (wetting with coloured solutions simulating bodily discharges) to be continuously observed. For example if the backsheet is unable to adequately resist liquid tranmission then the filter paper will become wet with the coloured solution and this can be observed in the mirror. The magnitude of the transmitted solution either as a weight or more preferable the size of the stain on the absorbent filter paper (simulating the panty) in addition to the time dependence of the transmission can be readily recorded.

The test solution is introduced to the test sample via a calibrated delivery system such as via a simple burette according to the desired test approach as detailed below. Once the pad has been loaded with the test solution a period of one (1) minute is allowed for the solution to be absorbed into the test sample so the topsheet (wearer facing surface) is free from pools of test solution.

Following the one minute wait the test sample is placed under a pressure of 70 g/cm$^2$ (grams per square centimeter) which is believed to reflect more stressful pressures that are nevertheless regularly obtained in-use. The test sample remains under the 70 g/cm$^2$ pressure for a period of at least 30 mins and measurements, for example the area of the coloured stain on the absorbent paper, are measured at 10 minute intervals. It is particularly important to measure over an extended period of time first because the mobility of some bodily discharges such as blood and second the process of diffusion through the microporous backsheet is relatively time consuming.

It is also important to understand the mechanism of wet-through failure and to ensure the exact test design is able to correctly assess this. For example a breathable backsheet with relatively large aperatures (> 200 µm) is more likely to fail due to a process of extrusion (such as when sitting the pressure exerted may force the liquid through the relatively large aperatures) which will happen relatively quickly on placing the test sample under pressure. Conversely, a porous material with smaller aperatures (<200 μm) is unlikely to fail due to simple extrusion but, rather via a process of simple diffusion or capillary driven diffusion. Such process are slow compared to extrusion processes.

Method 1a : High Gush Simulation

In this first test design we measure the imperviousness of the breathable backsheet under a high loading (sudden stressful gush of test solution) simulation. This in-use situation is the most difficult to control (it often occurs on standing up after a prolonged period of lying or sitting) because typically the absorbent core (or structure) requires a finite period of time to function and to adequately absorb and bind bodily discharges. For example, an absorbent core composed of cellulose fibres (airfelt, tissue) and absorbent gelling material requires several minutes before fluids can be adequately absorbed and tightly bound. Unbound discharges, occupying void or inter fibre spaces are very mobile and can quickly move to the backsheet to be extruded under pressure or transported through the backsheet via capillary forces.

The high gush simulation test is performed as detailed in the above general description under the following conditions for a typical sanitary napkin:

| Test Solution | Synthetic Urine + 1% Surfactant or AMF + 1% Surfactant |
|---|---|
| Gush Volume (ml)§ | For Sanitary napkin 10 ml. |
| Gush Rate (ml/min.) | 10 (i.e. 10 ml in 60 seconds) |
| Pressure applied: (after 1 min. wait) | 70 g/cm$^2$ |

Results reported as area of stain/Wet-Through in units of square cm (cm$^2$) at time elapsed = 10, 20, 30mins.

Method 1b : Repetitive Loading Simulation

In this test design the imperviousness of the backsheet under more typical loading conditions where bodily discharges occur is measured periodically and as repetitive steps rather than a single gush event. The repetitive loading simulation test as performed for a typical sanitary napkin is detailed according to the above general description with the following specific conditions:

Specifically the test sample is subjected to a 5 ml load of the test solution (see below) placed in the centre of the test sample. A period of 1 minute allows test liquid to be absorbed and the sample is placed under pressure for 5 minutes. After this period the size (area) of wet-through is measured and recorded. The pressure is immediately removed and the sample is again subjected to a second 5 ml load of test solution. Again after the 1 min wait for the liquid to be absorbed the sample (now containing 10 ml of test solution) is placed under pressure for 5 minutes. After this period the size (area) of wet-through is measured and recorded. The pressure is immediately removed the and the sample is again subjected to a third 5 ml load of test solution. Again after the 1 min wait for the liquid to be absorbed the sample (now containing 15 ml of test solution) is placed under pressure for 5 minutes and the stain size (wet-through) is again measured. The cycle is continued until the pad has been loaded to 20 ml. The pad is then left under the load for a further 30 mins and the final wet-through area is measured.

| Test Solution | Synthetic Urine+ 1% Surfactant or AMF + 1% Surfactant |
|---|---|
| Gush Volume (ml) | For Sanitary napkin repetitive stepwise 5 ml loadings. |
| Maximum Load§ | 20 ml |
| Loading Rate: (ml/min) | 2.5 (i.e. 5 ml in 2 minutes) |
| Pressure applied: (after 1 min. wait) | 70 g/cm$^2$ |

Results are typically reported as area of stain/Wet-Through in units of square cm (cm$^2$) after 5 mins
at loadings = 5, 10, 15 & 20 ml at time =5 mins after loading
and at time = 30 mins after 20 ml loading.

Test solution type and volumes utilised in the test methods.

It is important to match the test solution conditions to the product end use to be able to reliably assess potential breathable backsheet designs. Sanitary napkins are designed to contain menstrual discharges. These discharges can be quite varied for different women and may contain various levels of fatty acids and detergent type contaminants from

## EP 0 874 612 B2

daily hygenic practices (washing, laundering etc). These components are extremely mobile and may have very low surface tensions. Thus, the test fluid should contain surfactant as detailed below. The volumes of test solutions up to 10 ml for a gush is sufficiently high so that 95% of all in-use gush situations will fall within this range. Likewise a sanitary napkin in-use may be repetitively loaded up to 20 ml (95 % of all sanitary napkins fall in this range) but seldom higher. Typically a sanitary napkin will have 10 ml load (90% of all napkins) or less.

Although incontinence pads, baby diapers or pantyliners (napkins worn by a woman between the period or at the start/end of the period) have different requirements to those of sanitary napkins, a test solution closer to urine discharges can be used on a sanitary napkin. Nevertheless bodily contaminants (fatty acids, surfactants and detergent residues) are still found and it has been determined that the addition of surfactant to a synthetic urine solution correlates well to conditions found in use.

Since it is a common practice to use feminine hygiene products (sanitary napkins, pantyliners) also as a light incontinence device it also is appropriate to assess potential breathable backsheet materials or constructions also with a synthetic urine solution containing surfactant. The volumes again are chosen to reflect typical conditions that this application is likely to expose the products to. For diapers or more stressful incontinence applications the methods can be readily modified to simulate higher test solution loading volumes and rates of delivery.

Preparation of Test Solution Synthetic Urine + 1% Surfactant (UreaB/1%).

The test solution Synthetic Urine is first prepared in 10 kg master batch and smaller quantities are removed as required and surfactant added. Each 10 Kg UreaB batch is composed of the following components:

| Component: | Formula | Quantity/10Kg batch |
|---|---|---|
| Urea | | 200 g |
| Sodium Chloride | NaCl | 90 g |
| Magnesium Sulphate | $MgSO_4.7H_2O$ | 11 g |
| Calcium Chloride | $CaCl_2$ | 6 g |
| Distilled Water | $H_2O$ | 9693 g |

The 10 Kg master batch is prepared according to the procedure; For individual measurements typically 100 ml test solution UreaB/1% Surfactant is prepared by mixing 90 ml UreaB solution with 10 ml Surfactant. The UreaB/1% solution must be constantly mixed to ensure the components do not separate prior to usage.

Preparation of Test Liquid AMF: Artificial menstrual fluid + 1 % surfactant

Artificial Menstrual Fluid (AMF) is based on modified sheep's blood that has been modified to ensure it closely resembles human menstrual fluid in viscosity, electrical conductivity, surface tension and appearance. In addition we introduce a surfactant (1%) to this test fluid (supplied by by Pegesis/USA) to better reflect stress situations in which typical hygiene practice (and in some limited situations, dietary influences) may introduce additional surfactants or unexpected levels of, for example, fatty acids, that might lower the blood surface tension. Low surface tension menses is the biggest contributor to through backsheet wet-through failure on a breathable absorbent article such as a sanitary article.

**Reagents:**

1) Difibrinated sheep's blood is available from Unipath S.p.A {Garbagnate Milanese/Italy}.
2) Lactic Acid from J.T. Baker Holland Reagent Grade (85-95%w/w)
3) Potassium Hydroxide (KOH) from Sigma Chemical Co. USA, Reagent grade
4) Phosphate Buffer Saline Tablets from Sigma Chemical Co. USA, Reagent grade
5) Sodium Chloride from Sigma Chemical Co. USA, Reagent grade
6) Gastric Mucine from Sigma Chemical Co. USA,Type III (CAS 84082-644)
7) Distilled Water.

Step 1:

Prepare a 9 ± 1 % Lactic Acid Solution by dissolution of lactic acid powder and distilled water.

Step 2:

Prepare a 10% Potassium Hydroxide (KOH) solution by dissolving KOH powder into distilled water.

Step 3:

Prepare a Phosphate buffer solution buffered to pH = 7.2. by dissolving tablets as directed into 1 L distilled water.

Step 4:

Prepare and slowly heat to 45 t 5 °C a solution of the following composition:

o 460 t 5 ml of phosphate buffer solution
o 7.5 ± 0.5 ml of KOH solution

Step 5:

Prepare a Mucous Solution by slowly dissolution (with constant stirring) of approximately 30 grams of gastric mucine in the pre-heated (45 ± 5 °C) solution prepared in step 4. Once dissolved the solution temperature should be increased to between 50 - 80 °C and the mixture covered for approximately 15 mins. Turn the heat down to maintain a relatively constant temperature between 40 and 50 °C and continue to stir for a period of 2.5 hrs.

Step 6:

Remove the solution from the hot plate and allow the solution (from step 5) to now cool to less than 40 °C. Add 2.0 ml of the 10% lactic acid solution and mix thoroughly for 2 mins.

Step 7:

Place the solution in an Autoclave and heat to a temperature of 121 °C for 15 mins.

Step 8:

Allow the solution to cool to room temperature and dilute 1 to 1 with the difibrinated sheep's blood.
Following AMF preparation its viscosity, pH and conductivity are measured to ensure the blood characteristics lie in a range close to that of normal menstrual blood {(see reference H.J. Bussing "zur Biochemie de Menstrualblutes" Zbl Gynaec, 179,456 (1957)}. The viscosity should lie in the range of 7 to 8 (units cStK). The pH should lie in the range of 6.9 to 7.5 and the conductivity in the range 10.5 to 13 (units mmho). If the viscosity is not within the range specified above it should not be used and a new batch of AMF needs to be prepared. This may require adjustment to the quantity of gastric mucine used. Since this is a natural product its composition may alter from one lot to another.
For individual measurements typically 100 ml AMF test solution with surfactant is prepared by mixing 90 ml AMF solution (maintained at 25 °C) with 10 ml Surfactant. The AMF/1% surfactant solution must be constantly mixed to ensure the components do not separate priorto usage. The solution should be used only within 4 hours of preparation.

Examples:

Examples representative of the present invention are have been tested according to the test methods 1 a and 1 b and the results are detailed in Table 1. Each test sample was prepared under identical conditions in all regards except for the specific treatment applied to material either forming part of or in intimate fluid contact to the backsheet construction. For the test samples sanitary pads produced under the trade name "Always Ultra Normal" available from Procter & Gamble GmbH Schwalbach / Germany were manufactured according to normal manufacturing procedures except for a very low level of attachment of the backsheet to the total structure. This allowed the existing backsheet composed of an impervious (to both liquids and gasses) plastic film to be removed and substituted for an alternative breathable backsheet. The structure of the sanitary napkin was identical for all examples except for an additional surface treatment (lowering of the surface energy of one liquid/solid surface via silicone coating).

Example 1: (Reference)

In this example the plastic, impervious backsheet typically found on a sanitary napkin is replaced by a microporous film {supplied by Exxon Chemical Company, USA under the manufacturing code Exxaire XBF-100W} and positioned directly in contact with the absorbent core. No additional layers or additional surface treatments are applied.

Example 2:

Is an identical structure to that of example 1 except that the garment facing surface of the absorbent core tissue (lying in contact with the wearer facing surface of the aperatured microporous film {supplied by Exxon Chemical Company under the manufacturing code Exxaire XBF-100W} has been treated with a basis weight of about 6 $g/m^2$ thermally cured silicone. The silicone was manufactured by DOW Coming USA {sold under the trade name SYL-OFF 7048 Crosslinker/SYL-OFF 7677 Release coater (mix ratio 10% : 90%)}.

Example 3:

Is an identical structure to that of example 2 except the wearer facing surface of the microprous film {supplied by Exxon Chemical Company under the manufacturing code Exxaire XBF-100W} has been treated (printed) with a basis weight of about 2 $g/m^2$ thermally cured silicone. The silicone was manufactured by DOW Coming USA {sold under the trade name SYL-OFF 7048 Crosslinker/SYL-OFF 7677 Release coater (mix ratio 10%: 90%)}.

Example 4:

In this example an additional layer is present adjacent to the garment facing surface of the core and the wearer facing surface of the microporous film {supplied by Exxon Chemical Company under the manufacturing code Exxaire XBF-100W}. This layer is an aperatured film made of low Density PE {supplied by Tredegar Corporation, USA under the manufacturing code X-1522}. The wearer facing surface (lying in contact with the garment facing surface of the absorbent core) of the aperatured film has been additionally treated with a basis weight of about 2 $g/m^2$ thermally cured silicone. The silicone was manufactured by DOW Corning USA {sold under the trade name SYL-OFF 7048 Crosslinker/ SYL-OFF 7677 Release coater (mix ratio 10% : 90%).

Example 5:

In this example an additional layer has been added onto the garment facing surface of the simple microporous film {supplied by Exxon Chemical Company under the manufacturing code Exxaire XBF-1 00W}. This layer is composed of a laminated nonwoven {14MB/14SB manufactured by Corovin GmbH in Germany under the trade name MD 2005}. The laminated nonwoven is composed of 14 $g/m^2$ spunbond and 14 $g/m^2$ meltblown (wearer facing surface) has been additionally treated with a basis weight of about 4 gsm thermally cured silicone. The silicone was manufactured by DOW Coming USA {sold under the trade name SYL-OFF 7048 Crosslinker/SYL-OFF 7677 Release coater (mix ratio 10% : 90%)

Table 1:

| Wet-Through Testing Results for each example | | | | |
|---|---|---|---|---|
| Wet-Through Testing Example | Test Solution | Test Design Method | Wet-Through§ ($cm^2$) Untreated | Wet-Through§ ($cm^2$) Treated |
| 1 | UreaB/1% | 1a | 45 | - |
|  | AMF/1% | 1a | 55 | - |
|  | AMF/1% | 1b | 105 | - |
| 2 | UreaB/1% | 1a | 45 | 15 |
|  | AMF/1% | 1a | 55 | 25 |
|  | AMF/1% | 1b | 105 | 70 |
| 3 | UreaB/1% | 1a | 45 | 20 |

§ Wet Through values recorded at 30 mins after article loading.

Table 1: (continued)

| Wet-Through Testing Results for each example | | | | |
|---|---|---|---|---|
| Wet-Through Testing Example | Test Solution | Test Design Method | Wet-Through§ ($cm^2$) Untreated | Wet-Through§ ($cm^2$) Treated |
| | AMF/1% | 1a | 55 | 30 |
| | AMF/1% | 1b | 105 | 80 |
| 4 | UreaB/1% | 1a | 45 | zero |
| | AMF/1% | 1a | 55 | zero |
| | AMF/1% | 1b | 105 | zero |
| 5 | UreaB/1% | 1a | 45 | zero |
| | AMF/1% | 1a | 55 | zero |
| | AMF/1% | 1b | 105 | zero |

§ Wet Through values recorded at 30 mins after article loading.

## Fluid Contact Angle Determination: Method Nr. 2

The fluid contact angle test is a standard test to evaluate the nature of the interaction between a solid surface and a liquid droplet. The contact angle a droplet forms on a surface is a reflection of several interactions. The nature of the liquid, its surface tension, the nature of the solid and surface aberrations in addition to the nature of the liquid-solid interaction. Generally, a droplet on a rough surface typically exhibits a higher contact angle than a droplet on a smooth surface of the same chemical composition. If a droplet of water exhibits a contact angle greater than 90 degrees the surface is considered "hydrophobic" to the liquid. If the contact angle is less than 90 degrees then the surface is deemed "hydrophilic".

## **Basic Principle of the Methods:**

The contact angle a liquid makes on a surface can be measured by a variety of techniques. The technique utilised herein to measure contact angle is the "Wilhelmy Plate Technique". The principle of this technique is to suspend a sample of the solid over a water vessel and the sample is slowly lowered to a defined depth into the liquid water and then removed. The retarding force exerted by the water on the material sample on contact (zero immersion depth) is measured by a microbalance and the cosine of the contact angle is then determined from the equation:

$$F = ST \cdot P \cdot Cos \, \phi \, / \, g$$

Where

F =        Sample force at zero immersion depth as determined by the balance (mg)
P =        Perimeter of sample at the interface (cm)
ST =       Surface Tension (dynes cm)
CosØ =    Cosine of contact angle
g =        Acceleration due to gravity (at measuring location)

The equipment we have used to measure the contact angle is a Automated "Dynamic Contact Angle Analyser (model DCA-322)" manufactured by Cahn Instruments, Inc. Cerritos CA 90701-2275 USA. For each material assessed (see Table) a sample (24 mm x 30 mm) is prepared and attached to a glass slide as specified by in the equipment manual. Great care is made to ensure the material sample is not touched that might otherwise contaminate the material surface. Each material is measured 5 times to ensure accuracy of measurements and to minimise impact of manufacturing variability or surface irregularities.

Table 2:

| E.g. | Surface | Contact Angle Untreated* | Contact Angle Treated |
|------|---------|--------------------------|-----------------------|
| | Surface contact angles of liquid/solid materials and surfaces of the examples described above were measured. | | |
| 3 | Exxair XBF-1 00W Microporous Film Exxon Chemical Company, USA | 85 | 105 |
| 2 | Core Tissue Supplier Walkisoft Denmark | ~zero | 131 |
| 4 | LDPE Film Code X-1522 Supplier: Tredegar USA | 102 | 121 |
| 5 | Nonwoven MD2005 (14SM+14MB) Corovin GmbH, Piene, Germany | 117 | 140 |

The contact angle of a liquid on a surface and the ability of a porous material to transmit liquids either through capillary or extrusion processes is dependent on surface aberrations or surface structure, the nature of the liquid and how it interacts with the surface as well as the mechanism of transport. The test solution utilised in this test is distilled water with a high hydrophilicity and high surface tension. This leads to contact angles that are higher than those typically found or expected to be found with menstrual fluids or urine type discharges. Hence a contact angle greater than 90 degrees with water does not imply that the material pores will exert a negative capillary force on menstrual type discharges. However, an increase in the contact angle will work towards lowering the extent/efficiency of liquid transport (either capillary or extrusion based) through the test material.

**Claims**

1.  A disposable absorbent article (1) comprising a liquid pervious topsheet (24), an absorbent core (23) and a backsheet (20), said core (23) being intermediate said topsheet (24) and said backsheet (20), said backsheet (20) comprising a gas permeable, 2-dimensional, substantially planar apertured layer (21) and said core (23) comprising a fluid storage layer and said backsheet (20) comprising an outer layer,
    said core (23) and said backsheet (20) each comprising at least one layer, wherein each layer has a wearer facing surface and a garment facing surface and each of said surfaces of said layers has a fluid contact angle
    and said absorbent article having a lower portion extending from and including the garment facing surface of said fluid storage layer to and including the garment facing surface of said outer layer,
    wherein the wearer facing surface of at least one of said layers in said lower portion has a fluid contact angle greater than the fluid contact angle of the adjacent garment facing surface of an adjacent layer, and
    the garment facing surface of at least one of said layers in said lower portion has a fluid contact angle greater than the fluid contact angle of the wearer facing surface of said same layer.

2.  A disposable absorbent article (1) according to any one of the preceding claims, wherein at least one of the surfaces of said layers in said lower portion comprises a low surface energy material.

3.  A disposable absorbent article (1) according to claim 2, wherein said low surface energy material is selected from curable silicones, fluoropolymers, hydrocarbons or mixtures thereof.

4.  A disposable absorbent article (1) according to any one of the preceding claims, wherein either the garment facing surface or the wearer facing surface of said layer in said lower portion comprises at least 0.25g of a low surface energy material per square meter of said surface.

5.  A disposable absorbent article (1) according to any of the preceding claims, wherein said backsheet (20) comprises at least two layers.

6.  A disposable absorbent article (1) according to claim 5, wherein the second layer (22) of said backsheet (20) is located adjacent the wearer facing surface of said 2-dimensional layer (21).

7.  A disposable absorbent article (1) according to claim 5, wherein the second layer (22) of said backsheet (20) is located adjacent the garment facing surface of said 2-dimensional layer (21).

8. A disposable absorbent article (1) according to any one of the claims 5 to 7, wherein said second layer (22) is an apertured polymeric formed film or a 2 dimensional polymeric apertured layer or a fibrous layer.

9. A disposable absorbent article (1) according to any one of the preceding claims, wherein said core (23) comprises at least two portions, a first portion comprising said storage layer and a second portion comprising a fibrous layer, said fibrous layer being adjacent said backsheet.

10. A disposable absorbent article (1) according to any one of the preceding claims, wherein said wearer facing surface of said layer has a fluid contact angle at least 10° greater than the fluid contact angle of an adjacent surface.

11. A disposable absorbent article (1) according to claim 10, wherein said fluid contact angle is at least 20° greater than the fluid contact angle of an adjacent surface.

12. A disposable absorbent article (1) according to any one of the preceding claims, wherein said fluid contact angle of said garment facing surface of said storage layer is at least 90°.

13. A disposable absorbent article (1) according to claim 12, wherein said fluid contact angle of said surface is at least 100°.

14. A disposable absorbent article (1) according to any one of the preceding claims wherein said article (1) is a sanitary napkin or a panty liner.

15. A disposable absorbent (1) article according to claim 1, wherein said absorbent article (1) preferably has a continuous fluid contact angle gradient in said lower portion.

**Patentansprüche**

1. Absorbierender Einwegartikel (1) mit einer flüssigkeitsdurchlässigen Oberschicht (24), einem absorbierenden Kern (23) und einer Unterschicht (20), wobei sich der Kern (23) zwischen der Oberschicht (24) und der Unterschicht (20) befindet, wobei die Unterschicht (20) eine gasdurchlässige, zweidimensionale, im wesentlichen planar geöffnete Schicht (21) aufweist und der Kern (23) eine Fluidspeicherschicht aufweist und die Unterschicht (20) eine äußere Schicht aufweist,
wobei der Kern (23) und die Unterschicht (20) jeweils wenigstens eine Schicht aufweisen, wobei jede Schicht eine trägerseitige Oberfläche und eine wäscheseitige Oberfläche hat und jede der Oberflächen der Schichten einen Fluidkontaktwinkel hat
und der absorbierende Artikel einen unteren Bereich hat, der sich von der wäscheseitigen Oberfläche der Fluidspeicherschicht und diese einschließend zu der wäscheseitigen Oberfläche der äußeren Schicht und diese einschließend erstreckt,
wobei die trägerseitige Oberfläche wenigstens einer der Schichten in dem unteren Bereich einen Fluidkontaktwinkel von größer als dem Fluidkontaktwinkel der angrenzenden wäscheseitigen Oberfläche einer angrenzenden Schicht hat und
die wäscheseitige Oberfläche wenigstens einer der Schichten in dem unteren Bereich einen Fluidkontaktwinkel von größer als dem Fluidkontaktwinkel der wäscheseitigen Oberfläche der gleichen Schicht hat.

2. Absorbierender Einwegartikel (1) nach einem der vorstehenden Ansprüche, in welcher wenigstens eine der Oberflächen der Schichten in dem unteren Bereich ein Material geringer Oberflächenenergie aufweist.

3. Absorbierender Einwegartikel (1) nach Anspruch 2, in welchem das Material geringer Oberflächenenergie ausgewählt ist aus härtbaren Siliconen, Fluorpolymeren, Kohlenwasserstoffen oder Mischungen davon.

4. Absorbierender Einwegartikel (1) nach einem der vorstehenden Ansprüche, in welchem entweder die wäscheseitige Oberfläche oder die trägerseitige Oberfläche der Schicht in dem unteren Bereich wenigstens 0,25 g des Materials geringer Oberflächenenergie pro Quadratmeter der Oberfläche aufweist.

5. Absorbierender Einwegartikel (1) nach einem der vorstehenden Ansprüche, in welchem die Unterschicht (20) wenigstens zwei Lagen aufweist.

6. Absorbierender Einwegartikel (1) nach Anspruch 5, in welchem die zweite Lage (22) der Unterschicht (20) angrenzend an die trägerseitige Oberfläche der zweidimensionalen Schicht (21) angeordnet ist.

7. Absorbierender Einwegartikel (1) nach Anspruch 5, in welchem die zweite Lage (22) der Unterschicht (20) angrenzend an die wäscheseitige Oberfläche der zweidimensionalen Schicht (21) angeordnet ist.

8. Absorbierender Einwegartikel (1) nach einem der vorstehenden Ansprüche 5 bis 7, in welchem die zweite Lage (22) ein offener polymer geformter Film oder eine zweidimensionale polymere offene Schicht oder eine Faserschicht ist.

9. Absorbierender Einwegartikel (1) nach einem der vorstehenden Ansprüche, in welchem der Kern (23) wenigstens zwei Bereiche umfaßt, wobei ein erster Bereich die Speicherschicht aufweist und ein zweiter Bereich eine faserige Schicht aufweist, wobei die faserige Schicht an die Unterschicht angrenzt.

10. Absorbierender Einwegartikel (1) nach einem der vorstehenden Ansprüche, in welchem die trägerseitige Oberfläche der Schicht einen Fluidkontaktwinkel von wenigstens 10° größer als dem Fluidkontaktwinkel einer angrenzenden Oberfläche hat.

11. Absorbierender Einwegartikel (1) nach Anspruch 10, in welchem der Fluidkontaktwinkel wenigstens 20° größer als der Fluidkontaktwinkel einer angrenzenden Oberfläche ist.

12. Absorbierender Einwegartikel (1) nach einem der vorstehenden Ansprüche, in welchem der Fluidkontaktwinkel der wäscheseitigen Oberfläche der Speicherschicht wenigstens 90° beträgt.

13. Absorbierender Einwegartikel (1) nach Anspruch 12, in welchem der Fluidkontaktwinkel der Oberfläche wenigstens 100° beträgt.

14. Absorbierender Einwegartikel (1) nach einem der vorstehenden Ansprüche, in welchem der Artikel (1) eine Damenbinde oder eine Höscheneinlage ist.

15. Absorbierender Einwegartikel (1) nach Anspruch 1, in welchem der absorbierende Artikel (1) vorzugsweise einen kontinuierlichen Fluidkontaktwinkelgradienten im unteren Bereich hat.


**Revendications**

1. Article absorbant jetable (1) comprenant une feuille de dessus (24) perméable aux liquides, une âme absorbante (23) et une feuille de fond (20), ladite âme (23) étant placée entre ladite feuille de dessus (24) et ladite feuille de fond (20), ladite feuille de fond (20) comprenant une couche (21), perforée, essentiellement plane, bidimensionnelle, perméable aux gaz, ladite âme (23) comprenant une couche de stockage de fluides, et ladite feuille de fond (20) comprenant une couche extérieure,
   ladite âme (23) et ladite feuille de fond (20) comprenant chacune au moins une couche, où chaque couche a une surface côté utilisatrice et une surface côté vêtement, et chacune desdites surfaces desdites couches a un certain angle de contact de fluide,
   ledit article absorbant ayant une partie inférieure qui s'étend depuis la surface côté vêtement de ladite couche de stockage de fluides, et comprend cette dernière et comprend la surface côté vêtement de ladite couche extérieure,
   où la surface côté utilisatrice d'au moins l'une desdites couches dans ladite partie inférieure a un angle de contact de fluide supérieur à l'angle de contact de fluide de la surface côté vêtement contiguë d'une couche contiguë, et
   la surface côté vêtement d'au moins l'une desdites couches dans ladite partie inférieure a un angle de contact de fluide supérieur à l'angle de contact de fluide de la surface côté utilisatrice de ladite même couche.

2. Article absorbant jetable (1) selon l'une quelconque des revendications précédentes, dans lequel au moins l'une des surfaces desdites couches dans ladite partie inférieure comprend un matériau à faible énergie superficielle.

**3.** Article absorbant jetable (1) selon la revendication 2, dans lequel ledit matériau à faible énergie superficielle est choisi parmi les silicones durcissables, les polymères fluorés, les hydrocarbures, ou leurs mélanges.

**4.** Article absorbant jetable (1) selon l'une quelconque des revendications précédentes, dans lequel soit la surface côté vêtement soit la surface côté utilisatrice de ladite couche dans ladite partie inférieure comprend au moins 0,25 g d'un matériau à faible énergie superficielle par mètre carré de ladite surface.

**5.** Article absorbant jetable (1) selon l'une quelconque des revendications précédentes, dans lequel ladite feuille de fond (20) comprend au moins deux couches.

**6.** Article absorbant jetable (1) selon la revendication 5, dans lequel la deuxième couche (22) de ladite feuille de fond (20) est placée en contiguïté de la surface côté utilisatrice de ladite couche bidimensionnelle (21).

**7.** Article absorbant jetable (1) selon la revendication 5, dans lequel la deuxième couche (22) de ladite feuille de fond (20) est placée en contiguïté de la surface côté vêtement de ladite couche bidimensionnelle (21).

**8.** Article absorbant jetable (1) selon l'une quelconque des revendications 5 à 7, dans lequel ladite deuxième couche (22) est un film formé perforé, polymère, ou bien une couche perforée polymère, bidimensionnelle, ou encore une couche fibreuse.

**9.** Article absorbant jetable (1) selon l'une quelconque des revendications précédentes, dans lequel ladite âme (23) comprend au moins deux parties, une première partie comprenant ladite couche de stockage et une deuxième partie comprenant une couche fibreuse, ladite couche fibreuse étant contiguë à ladite feuille de fond.

**10.** Article absorbant jetable (1) selon l'une quelconque des revendications précédentes, dans lequel ladite surface côté utilisatrice de ladite couche a un angle de contact de fluide d'au moins 10° supérieur à l'angle de contact de fluide d'une surface contiguë.

**11.** Article absorbant jetable (1) selon la revendication 10, dans lequel ledit angle de contact de fluide est d'au moins 20° supérieur à l'angle de contact de fluide d'une surface contiguë.

**12.** Article absorbant jetable (1) selon l'une quelconque des revendications précédentes, dans lequel ledit angle de contact de fluide de ladite surface côté vêtement de ladite couche de stockage est d'au moins 90°.

**13.** Article absorbant jetable (1) selon la revendication 12, dans lequel ledit angle de contact de fluide de ladite surface est d'au moins 100°.

**14.** Article absorbant jetable (1) selon l'une quelconque des revendications précédentes, dans lequel ledit article (1) est une serviette hygiénique ou un protège-slip.

**15.** Article absorbant jetable (1) selon la revendication 1, dans lequel ledit article absorbant (1) a, de préférence, un gradient d'angle de contact de fluide continu dans ladite partie inférieure.

Fig. 1

## *Fig. 2*

## Fig. 3

A

110

112

## Fig. 4

A(b)   110   A(a)

113

115

b   dx   a